# EUROPEAN PATENT SPECIFICATION

(11) **EP 2 167 681 B1**
(45) Date of publication and mention of the grant of the patent: **16.01.2013**
(21) Application number: 08796097.7
(22) Date of filing: 03.07.2008
(51) Int. Cl.: C12Q 1/68, A61K 31/185

(54) **TREATMENT OF CARDIOVASCULAR DISEASE WITH SALICYLATES**
BEHANDLUNG VON HERZ-KREISLAUF-ERKRANKUNGEN MIT SALICYLATEN
TRAITEMENT DE MALADIE CARDIOVASCULAIRE AU MOYEN DE SALICYLATES

(30) Priority: 03.07.2007 US 958185 P; 07.05.2008 US 51228
(43) Date of publication of application: 31.03.2010
(73) Proprietor: Joslin Diabetes Center, Inc., Boston, MA 02115 (US)
(72) Inventor: SHOELSON, Steven, Natick, Massachusetts 01760 (US); GOLDFINE, Allison, B., Wayland, Massachusetts 01778 (US)
(74) Representative: Williams, Gareth Owen
(86) International application number: PCT/US2008/069177
(87) International publication number: WO 2009/006583

(56) References cited:
- US-A- 5 380 747
- US-A1- 2007 043 120
- US-B2- 6 844 149
- SILVER ROBERT J ET AL: "FDA-approved dose of salsalate improves glucose and lipid metabolism in type 2 diabetes" DIABETES, AMERICAN DIABETES ASSOCIATION, US, vol. 53, no. suppl. 2, 1 June 2004 (2004-06-01), page A161, XP009121671 ISSN: 0012-1797
- HUNDAL R S ET AL: "Mechanism by which high dose aspirin improves glucose metabolism in type 2 diabetes" JOURNAL OF CLINICAL INVESTIGATION, AMERICAN SOCIETY FOR CLINICAL INVESTIGATION, US LNKD- DOI:10.1172/JCI200214955, vol. 109, 1 January 2002 (2002-01-01), pages 1321-1326, XP002432772 ISSN: 0021-9738

## Description

### CROSS-REFERENCE TO RELATED APPLICATIONS

This application is related to U.S. Provisional Patent Applications Serial No. 60/958,185, filed on July 3, 2007, and 61/051,228, filed May 7, 2008.

### FEDERALLY SPONSORED RESEARCH OR DEVELOPMENT

This invention was made with Government support under Grant No. P50HL083813 awarded by the National Institutes of Health. The Government has certain rights in the invention.

### TECHNICAL FIELD

This invention relates to the treatment of atherosclerotic cardiovascular disease (ACVD) using an NF-kB inhibitor, e.g., non-acetylated forms of salicylate, e.g., salsalate and trilisate.

### BACKGROUND

Each year, cardiovascular disease (CVD) kills more Americans than cancer (United States Dept. of Health and Human Services, Center for Disease Control, Chronic Disease Overview. (1999)). Diseases of the heart alone caused 30% of all deaths (Id.). ACVD is the number one cause of death and disability in the United States and in most of Europe. In large part, the rise in ACVD is due to an increase in the risk factors for ACVD: abdominal obesity, Western diet and a sedentary life-style. Inflammation participates in the pathogenesis of cardiovascular disease (CVD) (Hansson, N. Engl. J. Med., 352(16):1685-1695 (2005); Hansson and Libby, Nat. Rev. Immunol. 6(7):508-519 (2006)).

### SUMMARY

The present inventors have discovered that NF-κB mediated inflammation is a "common soil" involved in the pathogenesis of a number of inflammatory diseases associated with one or more of: abdominal obesity, Western diet and a sedentary life-style, including atherosclerotic cardiovascular disease (ACVD). Therefore, the present methods include the systemic inhibition of NF-κB by administration of high doses of non-acetylated forms of salicylate, e.g., salsalate, trilisate, and other salicylate esters, amides and thioesters and salicylates having substituents on the ring (e.g., halogen, methyl, or halogenated methyl groups), for preventing or retarding the development or progression of cardiovascular diseases, e.g., atheroma formation.

In one aspect, the invention provides compositions for treating or preventing the development or progression of atherosclerotic cardiovascular disease (ACVD) in a subject. The methods include evaluating the subject for the presence of ACVD, or for the presence of one or more risk factors for ACVD; if the subject has or is at risk for ACVD, selecting the subject, and administering to the subject a therapeutically effective amount of an NF-kappaB inhibitor, e.g., a non-acetylated form of salicylate. In some embodiments, the methods of evaluating the subjects for the presence of ACVD include determining triglyceride levels, free fatty acid levels, cholesterol levels, and/or blood pressure, e.g., diastolic blood pressure, and selecting the subject based on the results of the determination. One or more other tests can also be used, e.g., angiogram (arteriogram); cholesterol test; chest x-ray; CT (computed tomography) scan; duplex scanning; echocardiogram; electrocardiogram (ECG or EKG); exercise stress test (cardiac stress test); intravascular ultrasound; MRI (magnetic resonance imaging) scan; PET (positron emission tomography) scan; and/or pharmacologic stress test.

In some embodiments, if the subject has ACVD, the methods include administering an NF-kappaB inhibitor. In some embodiments, the methods include administering about 2-5 g/day, e.g., about 3-4.5 g/day of the non-acetylated form of salicylate.

In some embodiments, the non-acetylated form of salicylate is salsalate or trilisate. In some embodiments, the ACVD is associated with elevated triglyceride levels, free fatty acid levels, total cholesterol levels, high LDL, and/or low HDL, and the methods include determining and optionally monitoring said levels. In some embodiments, the ACVD is associated with elevated diastolic blood pressure, and the methods include determining and optionally monitoring blood pressure, e.g., diastolic blood pressure, in the subject. Monitoring levels of an analyte or of blood pressure include determining the level at least twice, i.e., one to establish a base line level, and at least a second time to determine what effect the salsalate treatment is having on the analyte or blood pressure. In general, the determinations will be made at sequential time points, e.g., days or weeks apart, e.g., at least two weeks apart. In some embodiments, the methods include administering an amount of salsalate sufficient to reduce levels of triglycerides, free fatty acids, and/or cholesterol, or to reduce blood pressure, e.g., diastolic blood pressure.

In some embodiments, the subject is not suffering from pain, e.g., does not have severe or substantial pain.

Unless otherwise defined, all technical and scientific terms used herein have the same meaning as commonly understood by one of ordinary skill in the art to which this invention belong, Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

FIGs. 1A-C are a trio of images of aortas from Ldlr-/- mice fed chow (1A), Western diet (1B), or Western diet (1C), plus salicylate for 6 months. Aortas were removed and are displayed en face.
FIGs. 2A-D are photomicrographs of cross sections of the aortic roots of Ldlr-/- mice fed Western diet (2A-B) or Western diet plus salicylate (2C-D). Lipid in the fatty plaques is stained with oil red O (red in the original).
FIGs. 3A-B are bar graphs showing the results of quantification of lesion sizes in Ldlr-/-mice. 3A, data for 7 mice in each group treated as shown in FIGs. 1A-C. 3B, data for 6 mice/group as analyzed in FIGs. 2A-D. WD = Western diet; WD + Sal = Western diet plus salicylate.
FIGs. 4A-C are bar graphs showing Metabolic parameters in subjects prior to treatment (Pre, grey boxes) and after 2 wk treatment with 4.5 g/d (n = 7) or 3.0 g/d (n = 9) salsalate (Post, black boxes). Fasting data are displayed for (A) triglyceride (TG, mg/mlx0.0113 = mm01/L), (B) total cholesterol (mg/dl x 0.0259 = mm01/L), and (C) free fatty acids (FFA, mM). Data represent mean ± SEM; *P<0.05, **P<0.005.
FIGS. 5A-B are line graphs showing levels of free fatty acids (FFA) in response to the liquid meal for subjects treated with the highest tolerated salsalate dose (5A) or placebo (5B) either before (Pre, dashed grey lines) or after (Post, solid black lines) the 4 wk treatment with salsalate. Data represent mean ± SEM.

### DETAILED DESCRIPTION

The present inventors have discovered that NF-κB mediated inflammation is a "common soil" involved in the pathogenesis of inflammatory cardiovascular disease, including atherosclerosis. Therefore, the methods include the administration of high doses of non-acetylated forms of salicylate, e.g., salsalate, for preventing or retarding these diseases, e.g., preventing or retarding atheroma formation, even in the presence of continued high-risk behavior such as continued adherence to a high-fat Western diet and/or sedentary life style.

### General Methodology

The methods described herein, in general include the treatment or prevention of inflammation-mediated disorders that are associated with abdominal obesity, Western diet and/or a sedentary life-style by administering (e.g., orally administering) systemic doses of non-acetylated forms of salicylate, e.g., salsalate, diflunisal, or trilisate (e.g., choline-magnesium trilisate (CMT)). Salsalate is currently commercially available under the following brand names: Argesic®-SA; Disalcid®; Mono-Gesic®; Salflex®; and Salsitab®, and is presently prescribed to reduce the pain and inflammation (swelling) caused by arthritis or other inflammatory conditions. The conventional dosage is generally about 3000 mg daily, given orally in divided doses as follows: 1) two doses of two 750 mg tablets: 2) two doses of three 500 mg tablets/capsules; or 3) three doses of two 500 mg tablets/capsules. In some embodiments, the present methods generally include the administration of doses ranging from about 3000 to 4500 mg per day, but can also be lower, e.g., 2000 to 2500 mg per day, e.g., in subjects unable to tolerate the higher doses.

The methods described herein will generally include determining whether a subject has an inflammatory cardiovascular disease, or is at high risk for developing a inflammatory cardiovascular disease, and selecting the patient if they have or are at risk for such a disorder. In some embodiments of the methods described herein, the subjects are pain free, e.g., do not have joint pain, e.g., are not taking (or have not been prescribed) a non-acetylated forms of salicylate for the treatment of pain. In some embodiments, the subjects do not have diabetes or insulin resistance. Subjects who are at risk of ACVD include those who have one or more of the following risk factors: adherence to a Western diet, sedentary lifestyle, abdominal obesity, hypertension, dyslipidemia, or have elevated circulating levels of C-reactive protein CRP or other markers of inflammation, or low circulating levels of adiponectin,

The methods described herein include treating subjects who have, or are at risk of developing, cardiovascular disease, e.g., atherosclerosis. In some embodiments, the methods described herein include the systemic administration of high doses of non-acetylated salicylates in overweight (Body Mass Index (BMI) of 25-29) or obese (BMI of ≥ 30) non-diabetic subjects who are pain free, to treat patients with ACVD or to prevent the development of atherosclerotic cardiovascular disease, e.g., subjects who are at risk for developing such disease.

### Non-Acetylated Salicylates

The methods described herein can include the administration of a non-acetylated form of salicylate. A number of such compounds are known in the art, including salsalate, trilisate, and other salicylate esters, amides and thioesters and salicylates having substituents on the ring (e.g., halogen, methyl, or hologenated methyl groups). All of the compounds useful in the methods described herein will have NF-kappaB inhibitory activity in a standard *in vitro* assay.

### Salsalate

Salsalate (salicylsalicylic acid) is a dimer of salicylic acid; its structural formula is shown below.

### Chemical Structure:

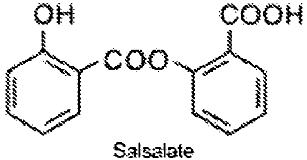

### Trilisate

Trilisate (choline magnesium trisalicylate) is freely soluble in water. The absolute structure of choline magnesium trisalicylate is not known at this time. Choline magnesium trisalicylate has a molecular formula of C₂₆H₂₉O₁₀NMg, and a molecular weight of 539.8. When dissolved in water, trilisate appears to form 5 ions (1 choline ion, 1 magnesium ion, and 3 salicylate ions).

### Other Non-Acetylated Salicylates

A number of other non-acetylated forms of salicylates are known in the art, including sodium salicylate, choline salicylate, magnesium salicylate, and diflunisal, as well as those that are ring-substituted forms, e.g., ortho, meta, or para-substituted forms; substituents include, e.g., halogen, methyl, or halogenated methyl groups. See, e.g., Soltani and De Brabander, Angewandte Chemie, 117(1), 1724-1727; Furst, Arth. & Rheum. 37(1),1-9 (1994); and Goodman and Gilman, The Pharmacological Basis of Therapeutics, 11th Edition, McGraw Hill (2005).

### Other NF-kappaB Inhibitors

In some embodiments, other NF-kappaB inhibitors can be used in place of or in addition to a non-acetylated form of salicylate, e.g., PS341, dehydroxymethyl-epoxyquinomicin (DHMEQ), include 2-chloro-N- [3, 5di (trifluoromethyl) phenyl]-4-(trifluoromethyl) pyrimidine-5-carboxamide (also known as SP-100030); 3,4-dihydro-4,4-dimethyl-2H-1,2-benzoselenazine (also known as BXT-51072); declopramide (also known as Oxi-104); dexlipotam; aryl, substituted or unsubstituted aralkyl, allyl, and substituted or unsubstituted, linear, branched, or cyclic alkyl esters of salicylic acid; nabumetone; sulindac sulfide; sulindac sulfone; sulfasalazine; and pharmaceutically acceptable salts thereof.

### Inflammation and atherogenesis.

An increasingly accepted sequence of events appears to link inflammation to the development and progression of atheroma formation (Blake and Ridker, J. Intern. Med. 252, 283-294 (2002); Libby, Circulation 104, 365-372 (2001)). For example, signs of inflammation accompany even the earliest accumulation of lipid within the arterial wall. Circulating leukocytes don't adhere to normal endothelium, but during an atherogenic diet the endothelium begins to express adhesion molecules that bind circulating leukocytes. These cell adhesion molecules include P- and E-selectins, ICAM-1, VCAM-1 and members of the immunoglobulin family. The selectins mediate transient rolling of the leucocytes along the endothelium while stronger attachments are mediated by ICAM-1 and VCAM-1 (Adams et al., Lancet 343, 831-836 (1994); Tedder et al., FASEB J. 9, 866-873 (1995)). Mice harboring genetic deletions of E- or P-selectin or ICAM-1 develop less atherosclerosis than normal mice (Collins et al., J. Exp. Med. 191, 189-194 (2000); Dong et al., J. Clin. Invest 102, 145-152 (1998); Mayadas et al., Cell 74, 541-554 (1993); Nageh et al., Arterioscler. Thromb. Vasc. Biol. 17, 1517-1520 (1997)).

Adherent inflammatory cells can migrate into the subendothelial space, where they contribute to the local inflammatory response and express a variety of chemoattractants that recruit additional cells as well as augmenting the differentiation of blood derived monocytes into macrophage foam cells and promoting the expression of modified lipoproteins on the macrophage surface (Gu et al., Mol Cell 2, 275-281 (1998); Qiao et al., Am. J. Pathol. 150, 1687-1699 (1997)). Cytokines and chemokines produced locally that participate in these processes include macrophage chemotactic protein (MCP-1) and the macrophage inflammatory proteins MIP-1α, MIP-1β, MIP-2, and MIP-3α. T-cell activation leads to expression of IFN-γ and lymphotoxin, which further amplify the proinflammatory state. Macrophages, endothelial cells and smooth muscles cells produce TNF-α (Barath et al., Am. J. Cardiol. 65, 297-302 (1990); Warner and Libby, J. Immunol. 142, 100-109 (1989)), which along with IFN-γ and IL-1β stimulates the local production of IL-6 in human atheroma (Rus et al., Atherosclerosis 127, 263-271 (1996); Seino et al., Cytokine 6, 87-91 (1994)). Paul Ridker has suggested that IL-6, possibly produced by atheromas, is the main hepatic stimulus for the acute phase reactant, C-reactive protein (CRP) (Blake and Ridker, J. Intern. Med. 252, 283-294 (2002)).

The CD40 ligand and its receptor CD40 are expressed by a wide variety of inflammatory cells. Ligation of CD40 triggers the expression of a host of pro-inflammatory mediators such as cytokines, chemokines, ICAM-1 and VCAM-1 and matrix metalloproteinases (MMPs) (Schonbeck and Libby, Circ. Res. 89, 1092-1103 (2001)). The latter can breakdown the collagen as well as tissue factor, an important mediator of thrombosis (Mach et al., Circulation 96, 396-399 (1997)).

As atherosclerotic lesions mature, the accumulation of foam cells leads to the formation of a lipid pool, rich in prothrombotic tissue factor. Smooth muscle cells produce collagen, which contributes to the strength of the fibrous cap and shields the circulating blood from the prothrombotic lipid pool. The synthesis and breakdown of collagen in the fibrous cap is dynamically mediated by inflammatory signals (Libby, Circulation 91, 2844-2850 (1995)). PDGF and TGF-β increase the rate of collagen production. Conversely, IFN-γ halts collagen synthesis by smooth muscle cells. Activated macrophages secrete matrix metalloproteinases, which degrade collagen and render the fibrous cap weak and prone to rupture (Libby, Circulation 104, 365-372 (2001)). Thus a dynamic balance is maintained between collagen synthesis and breakdown. If pro-inflammatory forces predominate, the fibrous cap may thin and eventually rupture, with release of the prothrombotic lipid pool into the lumen. This may herald the onset of an acute ischemic event.

### Selection of Subjects

The methods described herein include selecting subjects for treatment on the basis that they have, or are at risk of developing, ACVD. Methods for diagnosing ACVD are well known in the art, and include evaluating the subjects for the presence of ACVD include performing a diagnostic test, e.g., determining triglyceride levels, free fatty acid levels, cholesterol levels, and/or blood pressure, e.g., diastolic blood pressure, and selecting the subject based on the results of the diagnostic test. One or more other diagnostic tests can also be used, e.g., angiogram (arteriogram); cholesterol test; chest x-ray; CT (computed tomography) scan; duplex scanning; echocardiogram; electrocardiogram (ECG or EKG); exercise stress test (cardiac stress test); intravascular ultrasound; MRI (magnetic resonance imaging) scan; PET (positron emission tomography) scan; and/or pharmacologic stress test. The presence of detectable coronary calcium is one suitable indicator of the presence of ACVD.

Risk factors for ACVD are also well known and include high cholesterol in the blood (i.e., total blood cholesterol levels of 200 mg/dL); high low-density lipoprotein (LDL) in the blood (i.e., in subjects with heart disease or diabetes, LDL levels of 100 mg/dL or higher, or in the absence of other risk factors, LDL levels of 160 mg/dL or higher); a low level of high-density lipoprotein (HDL) in the blood (i.e., HDL levels of less than 40 mg/dL); high blood pressure (i.e., 140/90 mm Hg or higher); triglyceride levels (i.e., triglyceride levels above 150 mm/dL); tobacco smoke; diabetes mellitus; obesity (i.e., BMI of 25 or above); waist circumference (e.g., above 40 inches for men and 35 inches for women); waist-to-hip ratio (e.g., above about 1.0 for men or 0.90 or women, depending on subject, e.g., health status, age and race of the subject); inactive lifestyle (i.e., less than 30-60 minutes of physical activity on most days); age (i.e., age 45 for men and age 55 for women; and family history of heart disease (e.g., parents who developed coronary heart disease before age 55). In some embodiments, the methods include selecting a subject based on the presence of one or more of these risk factors. For example, the methods can include determining whether a subject has heart disease or diabetes, and evaluating LDL cholesterol levels, and selecting subjects who have one or both of heart disease or diabetes in addition to LDL levels of 100 mg/dL or higher. In some embodiments, the methods further include determining levels of C-reactive protein (CRP) in the subject, and if the levels are above a preselected threshold (see, e.g., Blake and Ridker, Arter. Thromb. Vasc. Biol. 22:1512 (2002)), selecting the subject.

The methods described herein can be administered in conjunction with one or more other treatments for ACVD, e.g., pharmacological treatment (e.g., anticoagulants, beta blockers; bile acid sequestrants; calcium channel blockers; ezetimibe; fibrates; glycoprotein IIb/IIIa receptor inhibitors; niacin (nicotinic acid); nitrates; platelet inhibitors; statins (HMG-CoA reductase inhibitors); and/or thrombolytics); surgeries and procedures (e.g., angioplasty; stent placement; coronary artery bypass surgery; carotid artery surgery; atherectomy); medical devices (e.g., stents; drug-eluting stents; cardiac angioplasty device; atherectomy device); lifestyle modification (e.g., control high blood pressure; control high blood cholesterol; prevent and manage diabetes mellitus; exercise; maintain a healthy weight; eat a healthy diet; quit or do not start smoking).

### Examples

The invention is further described in the following examples, which do not limit the scope of the invention described in the claims.

### Example 1: Salicylate-Mediated Inhibition of NF-κB Decreases Risk for Atherosclerosis

To evaluate the hypothesis that NF-κB activation by Western diet or obesity promotes atherogenesis as well as insulin resistance, experiments were conducted to determine whether anti-inflammatory salicylate therapy is a useful route for treating the metabolic syndrome.

Established models for studying atherosclerosis were used in these experiments, specifically the LDL receptor-deficient mice (Ldlr-/-) transgenic mice, which are on a C57B1/6 background. These mice were selected based on the magnitude of hypercholesterolemia (∼500 mg/dl in Ldlr-/-) and the impression that Ldlr-/- was an inflammation-driven model.

The mice were treated orally with salicylate, which provided therapeutic levels (>4 mg/dl) in the circulation.

The mice were fed normal rodent chow (4.5% fat g/g; Ralston Purina Co.) until the start of the experiment. Mice were housed in a VAF, pathogen-free transgenic facility on a 12 hour light-dark cycle with free access to food and water. Experiments were initiated when the mice were weaned at 4 weeks of age. Study groups included standard chow, plus or minus salicylate, and atherogenic, Western diet, plus or minus salicylate. The high fat (21% g/g), high cholesterol (0.15%), cholate-free diet was commercially available (Harlan Teklad 88137). The salicylate/diet trials proceeded for eight and 24 weeks. These time points were chosen to assess the impact of salicylate-mediated NF-κB inhibition on both early foam cell lesions and later, more complex atherosclerotic lesions.

### Quantitation of atherosclerosis in the mouse.

Several methods have been described to quantitate atherosclerosis lesion development in the mouse. Classically, lesions are visualized on cross sections of the aorta beginning at the level of the aortic sinus. A second method does not section the artery, but rather opens the vessel with a ventral incision and presents the entire surface of the thoracic and abdominal aorta en face. Both methods employ image analysis software to quantitate lesion size using digitized images. Both methods have been successfully employed, see, e.g., Bjorkbacka et al., Nat. Med. 10, 416-421 (2004). The system permits lesion area to be quantified, as well as for high-resolution morphologic analysis to be investigated. Quantification of atherosclerotic lesions was done by evaluation of lesion size in the aortic arch or descending thoracic and abdominal aorta.

At the trial termination, mice were anesthetized by i.p. injection with Avertin (0.4 mg/g body wt), and the arterial system was perfusion fixed with 10% formalin. Using an Olympus SZX12 dissecting microscope, the heart and arterial tree were removed and cleaned of peripheral fat. The heart was separated from the aortic root and embedded in optimal cutting temperature compound (OCT, Tissue-Tek). The heart was sectioned to the beginning of the aortic sinus, following which every third section (5 µm) was collected for analysis (400 µm total), stained with oil red O, and counterstained with hematoxylin.

For quantification of lesions in the descending aorta, the aorta was dissected free of all branching vessels, and stained, en face, with Sudan IV for 15 minutes, destained with 70% ethanol for 30 seconds, and stored in water. Stained aortas were incised ventrally from the aortic root to the bifurcation of the iliac arteries, laid flat on glass slides and covered by a coverslip. Images of stained aortas were acquired with a SPOT INSIGHT QE color digital camera with a Nikon MICRO-NIKKOR 60 mm f2.8 D macro lens using SPOT V3.5.7 software. Lesion areas were selected using IP LAB SPECTRUM software that utilizes pre-defined Sudan IV staining parameters to distinguish atherosclerotic regions within the aorta. Lesion areas were calculated as a percent of the total area of the whole aorta (or defined regions of the aorta such as the aortic arch, thoracic, abdominal, ileal areas). The tissue preparation, sectioning, and staining were performed as described in (Bjorkbacka et al., Nat. Med. 10, 416-421 (2004)).

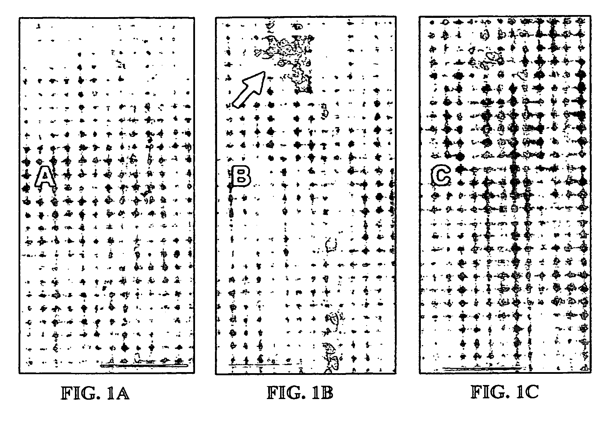

Shown in Figs. 1A-C is an example of the effect of salicylate in inhibiting plaque (fatty streak) formation in the descending aortas of Ldlr-/- mice. There was no detectable "plaque" in animals fed normal chow(Fig. 1A), yet a significant amount of plaque formation was seen in aortas of mice fed Western diet, particularly in the arch after the aorta exits the heart (Fig. 1B, arrow). Smaller lesions were seen throughout the thoracic and abdominal aorta (Fig. 1B). Salicylate treatment markedly reduced the plaque volume in mice on Western diet (Fig. 1C).

Frozen sections of the aortic sinus were also immunostained with antibodies against macrophage (F4/80 or MAC3), smooth muscle (alpha-actin) and endothelial (CD31) markers. Images of stained sections are acquired as described above and positively stained cells will be selected by IP Lab Spectrum software; immunoreactivity will be quantified as a percentage of total lesion area.

Images were acquired using computerized image analysis software linked to a Hitachi 3 CCD color video camera (Model HV-C20) mounted on a Nikon E600 microscope. The 4X PLANFLUOR objective was used to encompass the entire aortic sinus in the video image, while the 10X, 20X and 40X, objectives can be used for higher resolution detail. Magnification was calibrated using an in-view micrometer. Mean lesion area was determined from 12 digitally captured sections per mouse using a Wacom CINTIQ tablet to outline the lesion areas and IP Lab Spectrum software v.3.6 to calculate lesion area.

Statistical analyses were performed as follows. Power calculations with a threshold of detecting at least a 20% difference in lesion size predicted that 9 mice per experimental group would be required to show a statistically significant difference (using a two-tailed test with alpha = 0.05) with a 90% probability. Twelve mice of each sex were therefore used for each group, providing a small cushion for any unexpected animal losses. Should higher losses occur, only 7 mice per group are required to give an 80% probability of detecting a 20% difference.

Comparisons of atherosclerosis outcomes were analyzed by one-way analysis of variance (ANOVA) and Student's t-test. Data were plotted as mean lesion area ± SEM.

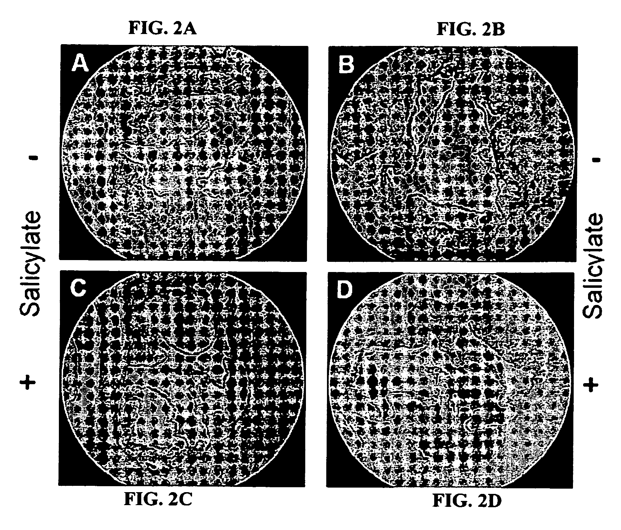

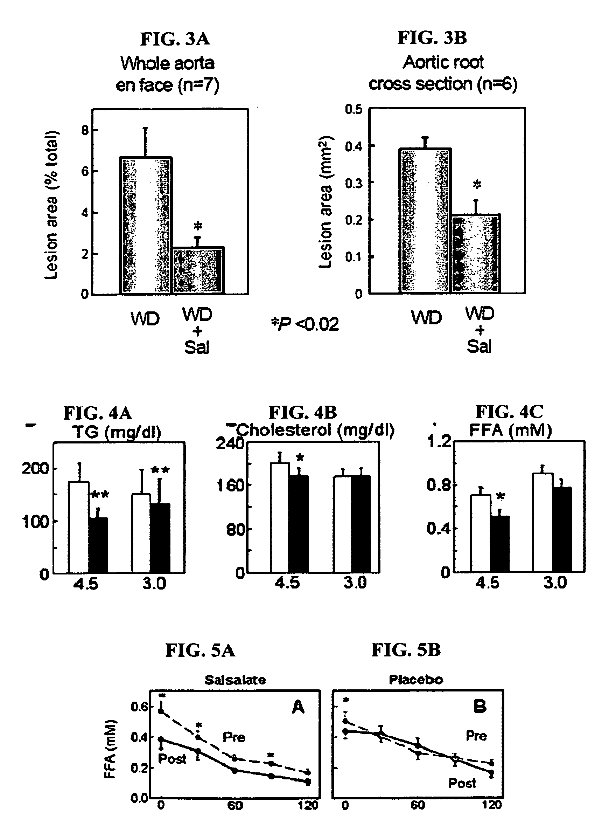

Data from the whole aorta en face preparations (Figs. 1A-C) and cross sectional analyses of the aortic root (Figs. 2A-D), from larger numbers of similarly treated animals, were quantified using these methods; the results are shown in Figs. 3A-B. The overwhelming conclusion was that the anti-inflammatory drug salicylate has a significant and beneficial effect in reducing plaque volume in this animal model of atherosclerosis.

These preclinical data support the use of salicylates as new therapeutic modalities in the treatment and/or prevention of the development or progression of cardiovascular disease, e.g., atheroma formation.

### Lipoprotein and lipid profiles of mouse serum.

Blood from overnight fasted mice is collected from the retro-orbital plexus, allowed to clot on ice, and centrifuged at 12000 rpm for 15 min at 4 oC. Serum is collected and concentrations of total cholesterol and triglycerides will be determined enzymatically (Sigma) as described (Eberhart et al., J. Clin. Endocrinol. Metab. 83, 836-846 (1998)). Plasma from animals in identical treatment groups is pooled and fractionated by fast pressure liquid chromatography (FPLC) on two Superose 6B columns linked in series (Id.). Cholesterol concentrations are measured on each fraction.

### Measurement of pro-inflammatory molecules by microarray and QRT-PCR in arterial lesions.

In addition to the studies described above, 6 mice of each genotype (LIKK/Ldlr-/-, FGK/Ldlr-/-, LISR/Ldlr-/-, FISR/Ldlr-/-, and Ldlr-/-) are fed a Western diet for 8 weeks and the aortic sinus will be isolated for analysis of inflammatory gene expression (Bjorkbacka et al (Nat. Med. 10, 416-421 (2004)). Mice are anesthetized by i.p. injection with Avertin (0.4 mg/g body wt), and the arterial system is perfused with RNA Later (Ambion). Using an Olympus SZX12 dissecting microscope, the aortic arch is separated from the heart and the mid thoracic aorta and placed in Trizol reagent (Invitrogen). Total RNA is isolated and 50 ng amplified using the MessageAmp aRNA kit (Ambion). RNA is differentially labeled with Cy3 and Cy5 dyes using the Micromax ASAP RNA labeling kit (Perkin Elmer) and hybridized to spotted oligonucleotide arrays. RNA from LIKK/Ldlr-/-, FIKK/Ldlr-/-, LISR/Ldlr-/-, FISR/Ldlr-/-, and Ldlr-/- arteries is compared to assess changes in aortic gene expression due to the activation or inhibition of NF-KB in fat or liver. Genes that are found to be differentially expressed in the aortic sinus by microarray analysis will be confirmed by QRT-PCR as we previously described (Cai et al., Cell 119, 285-298 (2004); Ceriello et al., Arterioscler. Thromb. Vasc. Biol. 24, 816-823 (2004)).

### Example 2: Salsalate-Mediated Reduction of Triglyceride, Free Fatty Acid and Cholesterol Levels in Human Plasma

To evaluate the hypothesis that Salsalate treatment has beneficiary effects on risk factors involved in the development of ACVD, experiments were conducted to determine the effect of Salsalate administration on triglyceride and free fatty acid levels in human plasma. Baseline characteristics of the population of subjects are shown in Table 1.

**TABLE 1. Subject characteristics for the open label 4.5 g/d and 3.0 g/d trials (mean ± SD). F, female; M, male; BMI, body mass index; FBG, fasting blood glucose (mg/dlx0.0555 = mmol/l).**

| Salsalate dose | 4.5 (g/d) | 3.0 (g/d) |
|---|---|---|
| Glucose Tolerance | 2 IGT, 5 T2D | 9 T2D |
| Gender | 5F, 2M | 6F, 3M |
| Age (yr) | 49 ± 9 | 51 ± 3 |
| BMI (kg/m²) | 32 ± 6 | 34 ± 3 |
| FBS (mmol/l) | 6.2 ± 2.4 | 11.1 ± 1.3 |
| HbA1c (%) | 6.3 ± 1.7 | 8.1 ± 0.5 |

The study used an open-label trial design of two weeks duration, with one dose at 4.5 g/d salsalate (1.5 g three-times daily) to match the dosage and duration used historically to improve glycosuria (Ebstein, Berliner Klinische Wochenschrift; 13:337-340 (1876); Gilgore, Diabetes, 9(5), 392-393 (1960); Hundal et al., J. Clin. Invest., 109(10), 1321-1326 (2002)) and the second at 3 g/d (1.5 g twice daily) as recommended to minimize side effects (Caraco Pharmaceuticals). Antihyperglycemic medications were discontinued one month prior to baseline. Subjects were instructed to monitor fasting blood glucose levels and with symptoms of hyperglycemia or hypoglycemia, and to avoid changing dietary or exercise habits. Subjects received misoprostol (200 µg orally four times daily), beginning three days prior and throughout the 2 wk treatment period, to compare results to those from the previously conducted high-dose aspirin study (Hundal et al., J. Clin. Invest. 109(10), 1321-1326 (2002)).

Plasma glucose was measured with a Beckman Instrument Glucose Analyzer. Immunoassays were performed in duplicate by commercial assay including RIA for insulin and C-peptide (Diagnostic Systems), adiponectin (LINCO), and ELISA for free fatty acids (WAKO), IL-6 (R&D) and sCD40L (Bender Medsystems) and nitrite (Cayman). hsCRP was analyzed by immunoturbidometry (WAKO). Glycated albumin was evaluated by Hitachi 911 lipid and protein analyzer and kits from AsahiKasei (Tokyo, JP). Deuterated glucose (6,6-[2H2]-glucose) enrichment was measured by gas chromatography-mass spectrometry analysis (Ferrannini, Metabolism, 37(3):287-301 (1988)). NF-κB DNA binding activity was assessed by ELISA using neutra-avidin plates (Pierce) pre-immobilized with 5'-biotin-labeled, double-stranded NF-κB binding oligonucleotides. Isolated peripheral blood mononuclear cells (PBMC) were lysed with Passive Lysis Buffer (Promega). DNA-bound NF-κB was detected using anti-p65 antibody (Santa Cruz sc-372), and quantified using HRP-conjugated anti-rabbit IgG.

Salsalate therapy was accompanied by decreases in fasting triglycerides (Fig. 4A), 40% (2.0 ± 0.4 vs 1.2 ± 0.2 mmol/L [174 ± 37 vs 105 ± 20 mg/dl], P = 0.007) at 4.5 g/d and 11% (1.7 ± 0.5 vs 1.5 ± 0.5 mmol/L [150 ± 47 vs 133 ± 46 mg/dl], P = 0.007) at 3.0 g/d. Other changes in fasting lipids were seen only at the higher dose, including reductions in total cholesterol by 12% (5.2 ± 0.5 vs 4.6 ± 0.4 mmol/L [201 ± 19 vs 177 ± 14 mg/dl], P = 0.04) (Fig. 4B) and non-esterified free fatty acids (FFA, Fig. 4C) by 28% (0.71 ± 0.05 vs 0.51 ± 0.05 mM, P = 0.02) with major fatty acid subtypes reduced equivalently.

In another study, a group of diabetic subjects participated in a double-masked, placebo-controlled parallel study design of one-month duration. At entry, participants were treated by lifestyle modification alone or in conjunction with either sulfonylurea or biguanide therapy (stably dosed for >4 wk and continued for the trial duration). After an overnight fast, subjects received a mixed meal tolerance test with blood samples collected at baseline and every 30 min for 2 hours (8 oz. BOOST™ High Protein Drink, Novartis; 240 calories; 41 g carbohydrate, 10 g protein, 4 g fat). Subjects were then randomized to receive placebo or salsalate (4.0 g/d), orally, divided twice daily. Misoprostol was not administered to subjects in this trial. Baseline characteristics of the population of subjects are shown in Table 2.

**TABLE 2. Subject characteristics for the placebo-controlled trial of salsalate at maximum tolerated dose (mean ± SD). F, female; M, male; BMI, body mass index; FBG, fasting blood glucose; Met, metformin; SFU, sulfonylurea; LS, lifestyle. Chol, cholesterol (mg/dlx0.0259 = mmol/L)**

| | **Salsalate** | **Placebo** | **P value** |
|---|---|---|---|
| **Age (yr)** | **51 ± 12** | **54 ± 8** | **0.6** |
| **Gender** | **3M, 5F** | **5M, 4F** | **0.5** |
| **BMI (kg/m²)** | **32.5 ± 6.4** | **31.5 ± 4.9** | **0.7** |
| **FBS (mmol/L)** | **7.5 ± 1.0** | **7.0 ± 1.4** | **0.4** |
| **HbA1c (%)** | **7.1 ± 1.2** | **6.7 ± 0.5** | **0.3** |
| **SBP (mm Hg)** | **125 ± 12** | **132 ± 15** | **0.3** |
| **DBP (mm Hg)** | **71 ± 4** | **78 ± 10** | **0.1** |
| **Chol (mmol/L)** | **3.9 ± 0.8** | **4.7 ± 0.7** | **0.04** |
| **Treatment** | **5 Met, 1 SFU, 2 LS** | **6 Met, 1 SFU, 2 LS** | **ns** |

In this trial salsalate was associated with a 33% reduction in fasting free fatty acid concentrations (0.57 ± 0.07 vs 0.38 ± 0.06 mM, P = 0.0009), which was sustained following a mixed meal challenge (Fig. 5A). This is consistent with previous findings in rodents treated with high-dose sodium salicylate or aspirin (Yuan et al., Science, 293(5535):1673-1677 (2001); Cai et al., Nat. Med., 11(2):183-190 (2005)) and humans treated with high-dose aspirin (Hundal et al., J. Clin. Invest., 109(10):1321-1326 (2002). This is an important result as patients with diabetes often have elevated circulating lipids that potentially contribute to both the pathophysiology of type 2 diabetes and its associated complications.

### Example 3: Salsalate Mediated Reduction of Blood Pressure in Human Test Cohort

To evaluate the hypothesis that Salsalate treatment has beneficiary effects on risk factors involved in the development of ACVD, experiments were conducted to determine the Salsalate mediated changes in blood pressure in the patients participating in the trial. Enrolled patients and study design were identical to the study described in Example 2.

Although blood pressure was already well controlled in these patients, there was an 8% drop in diastolic pressure in the group receiving 3.0 g/d (77 ± 4 vs 71 ± 5 mmHg, P = 0.02); the downward trends in systolic blood pressure in both groups and diastolic blood pressure at 4.5 g/d did not reach statistical significance (Table 3).

**TABLE 3: Indices for fasting subjects studied in the open label trials before salsalate therapy (Pre) and after 2 wk treatment (Post) (mean ± SEM) (HDL and LDL mg/dlx0.0259 = mmol/L), Cr, creatinine mg/dlx88.4 = µmol/L)**

| | **Salsalate 4.5 (g/d)** | | | **Salsalate 3.0 (g/d)** | | |
|---|---|---|---|---|---|---|
| | Pre | Post | P value | Pre | Post | P value |
| Weight (kg) | 88.6 ± 8.3 | 88.4 ± 8.4 | 0.8 | 93.7 ± 7.2 | 94.2 ± 7.1 | 0.2 |
| HDL (mmol/L) | 1.2 ± 0.1 | 1.1 ± 0.1 | 0.01 | 1.1 ± 0.1 | 1.1 ± 0.1 | 0.6 |
| LDL (mmol/L) | 3.2 ± 0.5 | 3.0 ± 0.4 | 0.2 | 2.7 ± 0.3 | 2.9 ± 0.3 | 0.2 |
| SBP (mm Hg) | 135 ± 4 | 128 ± 5 | 0.2 | 135 ± 6 | 125 ± 7 | 0.1 |
| DBP (mm Hg) | 75 ± 2 | 71 ± 4 | 0.3 | 77 ± 4 | 71 ± 5 | 0.02 |
| AST (U/L) | 25 ± 3 | 24 ± 4 | 0.6 | 20 ± 2 | 20 ± 2 | 0.7 |
| ALT (U/L) | 21 ± 1 | 24 ± 2 | 0.2 | 26 ± 4 | 22 ± 3 | 0.01 |
| Cr (µmol/L) | 67 ± 5 | 77 ± 5 | 0.005 | 68 ± 6 | 72 ± 6 | 0.04 |

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. A non-acetylated form of salicylate for use in a method of treating or preventing the development or progression of atherosclerotic cardiovascular disease (CVD) in a subject, the method comprising: evaluating the subject for the presence of ACVD, or for the presence of one or more risk factors for ACVD; if the subject has or is at risk for ACVD, selecting the subject, and administering to the subject a therapeutically effective amount of a non-acetylated form of salicylate.

2. The non-acetylated form of salicylate of claim 1, wherein if the subject has ACVD, the method includes administering about 3-4.5 g/day of the non-acetylated form of salicylate.

3. The non-acetylated form of salicylate of claim 1, wherein the non-acetylated form of salicylate is salsalate or trilisate.

4. The non-acetylated form of salicylate of claim 1, wherein the subject has ACVD.

5. The non-acetylated form of salicylate of claim 1, wherein the subject is not suffering from pain.

6. The non-acetylated form of salicylate of claim 1, wherein the subject does not have severe or substantial pain.

7. The non-acetylated form of salicylate of claim 1, further comprising determining a level of triglycerides in the subject.

8. The non-acetylated form of salicylate of claim 1, comprising monitoring levels of triglycerides in the subject, and administering an amount of salsalate sufficient to produce a decrease in triglyceride levels in the subject.

9. The non-acetylated form of salicylate of claim 1, further comprising determining a level of free fatty acids in the subject.

10. The non-acetylated form of salicylate of claim 1, further comprising monitoring levels of free fatty acids in the subject, and administering an amount of salsalate sufficient to produce a decrease in free fatty acid levels in the subject.

11. The non-acetylated form of salicylate of claim 1, further comprising determining a level of cholesterol in the subject.

12. The non-acetylated form of salicylate of claim 1, further comprising monitoring cholesterol levels in the subject, and administering an amount of salsalate sufficient to produce a decrease in cholesterol levels in the subject.

13. The non-acetylated form of salicylate of claim 1, further comprising determining blood pressure in the subject.

14. The non-acetylated form of salicylate of claim 1, further comprising monitoring diastolic blood pressure in the subject, and administering an amount of salsalate sufficient to produce a decrease in blood pressure in the subject.

15. The non-acetylated form of salicylate of claim 14, wherein diastolic blood pressure is reduced.

16. The non-acetylated form of salicylate of claim 1, wherein evaluating the subject for the presence of ACVD comprises performing one or more diagnostic tests for ACVD, and wherein the subject is selected if diagnostic test results indicate that the subject has ACVD.

17. The non-acetylated form of salicylate of claim 1, wherein evaluating the subject for the presence of one or more risk factors for ACVD comprises determining one or more of total blood cholesterol levels; low-density lipoprotein (LDL) levels in the blood; high-density lipoprotein (HDL) levels in the blood; blood pressure; or triglyceride levels, and wherein the subject is selected if the results of the determination indicate that the subject has an increased risk of developing ACVD.

## Patentansprüche

1. Nicht acetylierte Salicylatform zur Verwendung in einem Verfahren zur Behandlung oder Prävention der Entwicklung oder Progression einer atherosklerotischen kardiovaskulären Erkrankung (ACVD) bei einem Patienten, wobei das Verfahren Folgendes umfasst: Beurteilung des Patienten auf das Vorliegen einer ACVD, oder auf das Vorliegen von einem oder mehr Risikofaktor(en) für eine ACVD; wenn der Patient an einer ACVD leidet oder wenn bei ihm ein Risiko dafür vorliegt, Auswahl des Patienten und Verabreichung einer therapeutisch wirksamen Menge einer nicht acetylierten Salicylatform an den Patienten.

2. Nicht acetylierte Salicylatform nach Anspruch 1, wobei das Verfahren eine Verabreichung von ca. 3 bis 4,5 g/Tag der nicht acetylierten Salicylatform einschließt, wenn der Patient an einer ACVD leidet.

3. Nicht acetylierte Salicylatform nach Anspruch 1, wobei die nicht acetylierte Salicylatform Salsalat oder Trilisat ist.

4. Nicht acetylierte Salicylatform nach Anspruch 1, wobei der Patient an einer ACVD leidet.

5. Nicht acetylierte Salicylatform nach Anspruch 1, wobei der Patient nicht an Schmerzen leidet.

6. Nicht acetylierte Salicylatform nach Anspruch 1, wobei der Patient keine starken oder beträchtlichen Schmerzen hat.

7. Nicht acetylierte Salicylatform nach Anspruch 1, ferner umfassend die Bestimmung eines Triglyceridspiegels bei dem Patienten.

8. Nicht acetylierte Salicylatform nach Anspruch 1, umfassend die Überwachung der Triglyceridspiegel bei dem Patienten, und Verabreichung einer Salsalatmenge, die zur Herbeiführung einer Senkung der Triglyceridspiegel bei dem Patienten ausreicht.

9. Nicht acetylierte Salicylatform nach Anspruch 1, ferner umfassend die Bestimmung eines Spiegels freier Fettsäuren bei dem Patienten.

10. Nicht acetylierte Salicylatform nach Anspruch 1, ferner umfassend die Überwachung der Spiegel freier Fettsäuren bei dem Patienten, und Verabreichung einer ausreichenden Salsalatmenge zur Herbeiführung einer Senkung der Spiegel freier Fettsäuren bei dem Patienten.

11. Nicht acetylierte Salicylatform nach Anspruch 1, ferner umfassend die Bestimmung eines Cholesterinspiegels bei dem Patienten.

12. Nicht acetylierte Salicylatform nach Anspruch 1, ferner umfassend die Überwachung der Cholesterinspiegel bei dem Patienten, und Verabreichung einer Salsalatmenge, die zur Herbeiführung einer Senkung der Cholesterinspiegel bei dem Patienten ausreicht.

13. Nicht acetylierte Salicylatform nach Anspruch 1, ferner umfassend die Bestimmung des Blutdrucks bei dem Patienten.

14. Nicht acetylierte Salicylatform nach Anspruch 1, ferner umfassend die Überwachung des diastolischen Blutdrucks bei dem Patienten, und Verabreichung einer Salsalatmenge, die zur Herbeiführung einer Blutdrucksenkung bei dem Patienten ausreicht.

15. Nicht acetylierte Salicylatform nach Anspruch 14, wobei der diastolische Blutdruck reduziert wird.

16. Nicht acetylierte Salicylatform nach Anspruch 1, wobei die Beurteilung des Patienten auf das Vorliegen einer ACVD die Durchführung von einem oder mehr diagnostischen Test(s) auf eine ACVD umfasst, und wobei der Patient ausgewählt wird, wenn die diagnostischen Testergebnisse darauf hindeuten, dass der Patient an einer ACVD leidet.

17. Nicht acetylierte Salicylatform nach Anspruch 1, wobei die Beurteilung des Patienten auf das Vorliegen von einem oder mehr Risikofaktor(en) für eine ACVD die Bestimmung von einem oder mehr von den Gesamtcholesterinspiegeln im Blut; den Spiegeln von Lipoprotein niederer Dichte (LDL-Spiegeln) im Blut; den Spiegeln von Lipoprotein hoher Dichte (HDL-Spiegel) im Blut; dem Blutduck; oder den Triglyceridspiegeln umfasst, und wobei der Patient ausgewählt wird, wenn die Ergebnisse der Bestimmung darauf hindeuten, dass der Patient ein erhöhtes Risiko für die Entwicklung einer ACVD aufweist.

## Revendications

1. Forme non acétylée de salicylate pour une utilisation dans une méthode de traitement ou de prévention du développement ou de la progression d'une maladie cardiovasculaire athérosclérotique (ACVD) chez un sujet, la méthode comprenant: l'évaluation du sujet sur la présence d'un ACVD ou sur la présence d'un ou de plusieurs facteurs de risque pour une ACVD; si le sujet présente ou est à risque d'une ACVD, la sélection du sujet et l'administration au sujet d'une quantité thérapeutiquement efficace d'une forme non acétylée de salicylate.

2. Forme non acétylée de salicylate selon la revendication 1, dans laquelle, si le sujet présente une ACVD, la méthode inclut l'administration d'environ 3-4,5 g/jour de la forme non acétylée de salicylate.

3. Forme non acétylée de salicylate selon la revendication 1, où la forme non acétylée de salicylate est un salsalate ou un trilisate.

4. Forme non acétylée de salicylate selon la revendication 1, dans laquelle le sujet présente une ACVD.

5. Forme non acétylée de salicylate selon la revendication 1, dans laquelle le sujet ne souffre pas de douleur.

6. Forme non acétylée de salicylate selon la revendication 1, dans laquelle le sujet ne présente pas de douleur sévère ou substantielle.

7. Forme non acétylée de salicylate selon la revendication 1, comprenant en outre la détermination d'un niveau de triglycérides chez le sujet.

8. Forme non acétylée de salicylate selon la revendication 1, comprenant la surveillance de niveaux de triglycérides chez le sujet et l'administration d'une quantité de salsalate suffisante pour produire une diminution des niveaux de triglycérides chez le sujet.

9. Forme non acétylée de salicylate selon la revendication 1, comprenant en outre la détermination d'un niveau d'acides gras libres chez le sujet.

10. Forme non acétylée de salicylate selon la revendication 1, comprenant en outre la surveillance de niveaux d'acides gras libres chez le sujet et l'administration d'une quantité de salsalate suffisante pour produire une diminution des niveaux d'acides gras libres chez le sujet.

11. Forme non acétylée de salicylate selon la revendication 1, comprenant en outre la détermination d'un niveau de cholestérol chez le sujet.

12. Forme non acétylée de salicylate selon la revendication 1, comprenant en outre la surveillance de niveaux de cholestérol chez le sujet et l'administration d'une quantité de salsalate suffisance pour produire une diminution des niveaux de cholestérol chez le sujet.

13. Forme non acétylée de salicylate selon la revendication 1, comprenant en outre la détermination de la tension artérielle chez le sujet.

14. Forme non acétylée de salicylate selon la revendication 1, comprenant en outre la surveillance de la tension artérielle diastolique chez le sujet et l'administration d'une quantité de salsalate suffisante pour produire une diminution de la tension artérielle chez le sujet.

15. Forme non acétylée de salicylate selon la revendication 14, dans laquelle la tension artérielle diastolique est réduite.

16. Forme non acétylée de salicylate selon la revendication 1, dans laquelle l'évaluation du sujet sur la présence d'une ACVD comprend la réalisation d'un ou de plusieurs essais diagnostiques pour une ACVD et dans laquelle le sujet est sélectionné si les résultats des essais diagnostiques indiquent que le sujet présente une ACVD.

17. Forme non acétylée de salicylate selon la revendication 1, dans laquelle l'évaluation du sujet sur la présence d'un ou de plusieurs facteurs de risque pour une ACVD comprend la détermination d'un ou de plusieurs de niveaux de cholestérol total dans le sang; de niveaux de lipoprotéines de basse densité (LDL) dans le sang; de niveaux de lipoprotéines de haute densité (HDL) dans le sang; de tension artérielle; ou de niveaux de triglycérides, et dans laquelle le sujet est sélectionné si les résultats de la détermination indiquent que le sujet présente un risque accru de développer une ACVD.
